(19) Europäisches Patentamt / European Patent Office / Office européen des brevets

(11) **EP 4 640 203 A1**

(12) **EUROPEAN PATENT APPLICATION**
published in accordance with Art. 153(4) EPC

(43) Date of publication:
**29.10.2025 Bulletin 2025/44**

(21) Application number: **23907875.1**

(22) Date of filing: **22.12.2023**

(51) International Patent Classification (IPC):
**A61K 8/365** (2006.01)   **A61K 8/34** (2006.01)
**A61Q 19/00** (2006.01)

(52) Cooperative Patent Classification (CPC):
**A61K 8/34; A61K 8/365; A61Q 19/00**

(86) International application number:
**PCT/KR2023/021439**

(87) International publication number:
**WO 2024/136592 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.12.2022  KR 20220181648**

(71) Applicant: **LG H&H Co., Ltd.**
**Seoul 03184 (KR)**

(72) Inventors:
• **KWON, Koo Chul**
**Seoul 07795 (KR)**
• **WON, Jong Gu**
**Seoul 07795 (KR)**
• **KIM, Mi Sun**
**Seoul 07795 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **SKIN IMPROVEMENT COMPOSITIONS CONTAINING CARNITINE SALICYLATE (CA-SA) AS ACTIVE INGREDIENT**

(57)   The present invention relates to cosmetic, pharmaceutical, and quasi-drug compositions containing carnitine salicylate (CA-SA) and magnolol as active ingredients, which show excellent effects of alleviating acne, alleviating sebum secretion, antibacterial action, and skin soothing, and can be useful in the prevention, amelioration, or treatment of skin inflammatory diseases.

[FIG.2]

**Vehicle applied**          **CA-SA + Magnolol**

Before

After 4 weeks

EP 4 640 203 A1

**Description**

[Technical Field]

The present invention relates to a composition for improving skin comprising carnitinesalicylate as an active ingredient.

[Background Art]

Acne is a common condition among adolescents, affecting approximately 85% of adolescents, but some may continue to have acne or develop it again after becoming adults.

Acne may be classified into non-inflammatory acne such as comedones and inflammatory acne such as papules, pustules, and nodules, and in particular, inflammatory acne may cause physical and mental pain by leaving behind erythema, pigmentation, and acne scars. Therefore, continuous research is required on the causes and treatment of acne.

The main causes of acne include excessive sebum production, keratosis and occlusion of hair follicles, C. *acnes* bacteria, and inflammation around the hair follicles. Acne treatment methods often include topical or systemic antibiotics and retinoids, and combination treatments may include chemical peels, hormone therapy, and laser therapy.

However, these treatments are often accompanied by side effects such as microbial resistance to antibiotics, stomatitis, skin irritation, and teratogenicity of retinoids. Therefore, there is still a need for research on acne treatment methods.

Salicylic acid, known as β-hydroxy acid (β-acid, BHA), is a metabolite of acetylsalicylic acid that exhibits analgesic and antirheumatic activities. Salicylic acid has been used for acne patients due to its antibacterial effects. However, repeated use of salicylic acid can cause skin irritation and dryness, so it is recommended to use it in cosmetics at a pH of 3.5 or higher and at a concentration of 3.0% or less. In addition, when applied in a weakly acidic condition in an ionized form such as salicylate, skin irritation may be reduced, but there is a limitation that skin permeability and efficacy decrease, making it difficult to experience high efficacy without skin irritation.

It has recently been discovered that a eutectic mixture of salicylic acid and carnitine has the effects of alleviating acne and promoting keratin peeling (Patent Document 1). As the existence of this eutectic mixture becomes known, research is needed on methods of utilizing it to provide better effects to the skin.

[Related Art Documents]

[Patent Document]

(Patent Document 1) Korea Patent Publication No. 2022-0126254

[Disclosure]

[Technical Problem]

Against the above-described background, the present inventors confirmed that when carnitine salicylate (CA-SA) and magnolol are used in combination, various effects for improving skin, such as antibacterial action, acne and inflammation alleviation, skin erythema recovery, and sebum secretion alleviation are achieved, thereby completing the present invention.

Therefore, an object of the present invention is to provide a composition for improving skin, including carnitine salicylate (CA-SA) and magnolol as active ingredients.

[Technical Solution]

To solve the above-described problem, the present invention provides a cosmetic, pharmaceutical, or quasi-drug composition including carnitine salicylate (CA-SA) and magnolol as active ingredients.

As another aspect, there is provided a method for improving skin or a method for preventing, ameliorating, or treating an inflammatory skin disease, comprising a step of applying a composition including CA-SA and magnolol to the skin.

As still another aspect, there is provided a use of CA-SA and magnolol for preparing a composition for improving skin or a composition for preventing, ameliorating, or treating an inflammatory skin disease.

Hereinafter, the present invention will be described in detail.

The present invention provides a cosmetic composition for improving skin, including CA-SA and magnolol as active ingredients.

As another aspect, there is provided a method of skin improvement, including a step of applying a composition including CA-SA and magnolol to the skin.

As still another aspect, there is provided a use of CA-SA and magnolol for preparing a cosmetic composition for skin improvement. In the present invention, the carnitine may be 4-trimethylamino-3-hydroxybutyric acid, and L-carnitine, DL-carnitine, a hydrochloride thereof, and derivatives thereof may be used. Preferably, it may be L-carnitine.

In the present invention, a eutectic mixture in the form of CA-SA in which salicylic acid ion-pairs with carnitine may be prepared and used, rather than using salicylic acid in the form of a salt.

The eutectic mixture is a mixture of two or more solid or liquid substances where two components having high melting points are mixed to form a complex by polar intermolecular dipole-dipole attraction, dipole-induced dipole attraction, intermolecular hydrogen bonding, or van der Waals interaction. In other words, the bonding of the eutectic mixture involves the bonding (formation of the eutectic mixture) between molecules that is formed based on hydrogen bonding and dipole-dipole bonding due to partial charges of molecules while the unique molecular structure of the individual molecules is maintained.

In addition, the eutectic mixture may form a eutectic mixture by bonding compounds having polarity together. The compounds having polarity may be polar molecules or charged molecules. In addition, there must be a polarity difference between the compounds having polarity, and identical compounds may not form a eutectic mixture.

For the eutectic mixture, the ratio of substances required for the eutectic mixture may be determined according to the charge of the molecules of the components forming the eutectic mixture, the type and number of functional groups of the molecules, and the polarity of the molecules or functional groups. In addition, the ratio of substances required for the eutectic mixture may be determined according to the size or similarity of the molecules.

In the present invention, the ratio of substances required for the CA-SA eutectic mixture may be determined according to the charge and functional group of the carnitine and salicylate molecules and the polarity of the molecules or functional groups. The CA-SA may be formed by mixing carnitine and salicylate at a molar ratio of carnitine:salicylate=1 to 6:2, for example, 6:2, 5:2, 4:2, 3:2, 2:2, 1:2, 5.5:2, 4.5:2, 3.5:2, 2.5:2, or 1.5:2. Carnitine and salicylate may form a eutectic mixture by dipole-to-dipole bonding due to partial charges of specific functional groups, and may form a eutectic mixture at the molar ratio in the above-described range through their chemical structures. When the molar ratio is outside of the above-described range, the low-temperature stability of carnitine may decrease, and salicylate may precipitate at a low temperature, as the remaining salicylate molecules that fail to ion-pair with carnitine exceed their solubility in water.

In the present invention, magnolol is a lignin with a molecular weight of 266.33 and a molecular formula of $C_{18}H_{13}O_2$, which is present in the dried bark, root bark, and the like *of Magnolia officinalis* Rehd. EtWils, a plant of the family Magnoliaceae, and is known to have antibacterial, antifungal, and antioxidant activities.

As a result of evaluating the efficacy of combined use of CA-SA and magnolol *in vitro* and *in vivo,* it was confirmed that it can exhibit excellent efficacy in antibacterial efficacy against acne bacteria, sebum secretion alleviation, inhibition of inflammatory factor expression, keratin softening efficacy, skin erythema recovery efficacy, and anti-acne activity. In particular, it can exhibit a significantly increased effect compared to when CA-SA and magnolol are used alone.

In the present invention, combined use of CA-SA and magnolol can exhibit an effect that is more improved than the original efficacy of each component. Specifically, the effect enhancement rate due to the combined use relative to the single use of CA-SA and magnolol may be 30% or more, 50% or more, 60% or more, 70% or more, 100% or more, 120% or more, 140% or more, 200% or more, 250% or more, 300% or more, 400% or more, or 600% or more.

In one embodiment of the present invention, CA-SA may be included in an amount of 0.01 to 20 parts by weight, for example, 0.01 to 20 parts by weight, 0.02 to 20 parts by weight, 0.03 to 20 parts by weight, 0.05 to 20 parts by weight, 0.15 to 20 parts by weight, 0.2 to 20 parts by weight, 0.5 to 20 parts by weight, 1 to 20 parts by weight, 1.5 to 20 parts by weight, 0.01 to 10 parts by weight, 0.02 to 10 parts by weight, 0.03 to 10 parts by weight, 0.05 to 10 parts by weight, 0.15 to 10 parts by weight, 0.2 to 10 parts by weight, 0.5 to 10 parts by weight, 1 to 10 parts by weight, 1.5 to 10 parts by weight, 0.01 to 5 parts by weight, 0.02 to 5 parts by weight, 0.03 to 5 parts by weight, 0.05 to 5 parts by weight, 0.15 to 5 parts by weight, 0.2 to 5 parts by weight, 0.5 to 5 parts by weight, 1 to 5 parts by weight, 1.5 to 5 parts by weight, 0.01 to 1.5 parts by weight, 0.02 to 1 part by weight, 0.03 to 0.5 parts by weight, or 0.05 to 0.2 parts by weight based on 100 parts by weight of the total composition, but is not limited thereto.

When the CA-SA according to the present invention is included in an amount of less than 0.01 parts by weight of the total composition, sufficient acne alleviation, inflammation alleviation, and erythema alleviation effects may not be expected, and when it is included in excess of 20 parts by weight, unwanted reactions such as allergies may occur or problems with skin safety may occur, and the above-described range prevents these problems.

In one embodiment of the present invention, magnolol may be included in an amount of 0.0000001 to 10 parts by weight, 0.000001 to 10 parts by weight, 0.0001 to 10 parts by weight, 0.0005 to 10 parts by weight, 0.001 to 10 parts by weight, 0.0000001 to 5 parts by weight, 0.000001 to 5 parts by weight, 0.0001 to 5 parts by weight, 0.0005 to 5 parts by weight, 0.001 to 5 parts by weight, 0.0000001 to 2 parts by weight, 0.000001 to 2 parts by weight, 0.0001 to 2 parts by weight, 0.0005 to 2 parts by weight, 0.001 to 2 parts by weight, 0.0000001 to 1 part by weight, 0.000001 to 1 part by weight, 0.0001 to 1 part by weight, 0.0005 to 1 part by weight, 0.001 to 1 part by weight, 0.0000001 to 0.5 parts by weight, 0.000001 to 0.5 parts by weight, 0.0001 to 0.5 parts by weight, 0.0005 to 0.5 parts by weight, 0.001 to 0.5 parts by weight, 0.000001 to 0.001 parts by weight, or 0.0001 to 0.0005 parts by weight based on 100 parts by weight of the total composition.

When magnolol according to the present invention is included in an amount of less than 0.0000001 parts by weight based on 100 parts by weight of the total composition, sufficient acne alleviation, inflammation alleviation, and erythema alleviation effects may not be expected, and when it is included in excess of 10 parts by weight, it may not be sufficiently dissolved in the composition.

In the present invention, magnolol may be included in the composition in the form of a plant extract containing magnolol, for example, a magnolia extract or a *Magnolia officinalis* extract. As an example, it may be included as a Japanese magnolia bark extract. When magnolol is included in the form of a plant extract containing magnolol, the plant extract may be included in the composition in an amount that satisfies the above-described magnolol content. For example, a plant extract containing magnolol may be included in an amount of 0.00001 to 10 parts by weight, 0.0001 to 10 parts by weight, 0.01 to 10 parts by weight, 0.05 to 10 parts by weight, 0.1 to 10 parts by weight, 0.00001 to 5 parts by weight, 0.0001 to 5 parts by weight, 0.01 to 5 parts by weight, 0.05 to 5 parts by weight, 0.1 to 5 parts by weight, 0.00001 to 2 parts by weight, 0.0001 to 2 parts by weight, 0.01 to 2 parts by weight, 0.05 to 2 parts by weight, 0.1 to 2 parts by weight, 0.00001 to 1 part by weight, 0.0001 to 1 part by weight, 0.01 to 1 part by weight, 0.05 to 1 part by weight, 0.1 to 1 part by weight, 0.00001 to 0.5 parts by weight, 0.0001 to 0.5 parts by weight, 0.01 to 0.5 parts by weight, 0.05 to 0.5 parts by weight, 0.1 to 0.5 parts by weight, 0.0001 to 0.1 parts by weight, or 0.01 to 0.05 parts by weight based on 100 parts by weight of the composition.

In one embodiment of the present invention, the weight ratio of CA-SA and magnolol may be from 500000 to 20:1. For example, it may be 500000 to 20:1, 300000 to 30:1, 5000 to 50:1, 3500 to 60:1, 2000 to 70:1, 500 to 100:1, 400 to 100:1, 300 to 100:1, 400 to 200:1, 300 to 200:1, 280 to 230:1, or 250:1.

In the present invention, improving skin may be one or more selected from the group consisting of acne alleviation, sebum secretion alleviation, skin soothing, antibacterial action, skin erythema alleviation, acne inflammation amelioration, and keratin peeling promotion.

In the present invention, "acne alleviation" may mean suppressing, inhibiting, or ameliorating skin acne symptoms, and "acne inflammation amelioration" may mean suppressing, alleviating, or ameliorating inflammation caused by acne bacteria or acne symptoms.

In the present invention, "keratin peeling" may mean that keratin accumulated in the keratin layer of the skin is dropped, removed, or softened from the keratin layer of the skin.

In the present invention, "antibacterial action" may mean suppressing, controlling, or preventing the growth or proliferation of bacteria, fungi, or the like (e.g., acne bacteria) or microorganisms.

In the present invention, the cosmetic composition may be in the form of a general emulsified formulation and a solubilized formulation. For example, it may have the formulation of a toner such as an emollient toner or a nourishing toner, an emulsion such as a facial lotion or a body lotion, a cream such as a nourishing cream, a moisturizing cream, an eye cream, or the like, an essence, a cosmetic ointment, a balm, a spray, a gel, a pack, a sunscreen, a makeup base, a foundation such as a liquid type, a solid type or a spray type, a powder, a makeup remover such as a cleansing cream, a cleansing lotion, or a cleansing oil, a cleansing foam, a soap, a body wash, or the like.

In addition to the composition of the present invention, the cosmetic may contain adjuvants commonly used in the field of cosmetology, such as fatty substances, organic solvents, solubilizers, thickening agents, gelling agents, emollients, antioxidants, suspending agents, stabilizers, foaming agents, fragrances, surfactants, water, ionic or nonionic emulsifiers, fillers, sequestering agents, chelating agents, preservatives, vitamins, blocking agents, humectants, essential oils, dyes, pigments, hydrophilic or lipophilic active agents, lipid vesicles, or any other ingredients commonly used in cosmetics.

The cosmetic formulation may include the composition of the present invention at a relatively high concentration in the case of wash-off type cosmetics such as makeup removers and cleansers, whose active ingredient remains on the skin for a short time. On the other hand, leave-on type cosmetics such as toners, emulsions, creams, and essences, whose active ingredient remains on the skin for a long time, may include the composition of the present invention at a lower concentration than the wash-off type cosmetics.

Each of the above-described ingredients included in the cosmetic composition according to the present invention may be included in the cosmetic composition of the present invention within a range that does not exceed the maximum usage amount stipulated in the cosmetic safety standards of each country.

In addition, the present invention provides a pharmaceutical composition for preventing or treating an inflammatory skin disease, including CA-SA and magnolol as active ingredients.

As yet another aspect, there is provided a method of preventing or treating an inflammatory skin disease, including a step of administering a composition including CA-SA and magnolol to a subject in need thereof.

As yet another aspect, there is provided a use of CA-SA and magnolol for preparing a pharmaceutical composition for preventing or treating an inflammatory skin disease.

As yet another aspect, there is provided a use of CA-SA and magnolol for preparing an acne therapeutic agent.

**In** the present invention, the inflammatory skin disease may be one or more selected from the group consisting of atopic dermatitis, seborrheic dermatitis, and acne, more specifically acne.

**In** the present invention, the term "prevention" refers to any act of suppressing or delaying a target symptom by administering the composition of the present invention.

**In** the present invention, the term "amelioration" refers to any act of ameliorating or beneficially changing a target symptom by administering the composition of the present invention compared to the symptom before the administration.

**In** the present invention, the term "treatment" refers to any act of alleviating, relieving, stopping or reversing a target symptom by administering the composition of the present invention.

The term "pharmaceutical composition" refers to a composition used for the purpose of diagnosing, treating, alleviating, treating, or preventing a disease of any animal, including a human.

The pharmaceutical composition of the present invention may be used alone or in combination with other pharmaceutically active compounds that exhibit preventive, ameliorating or therapeutic effects on aging, or may be used in an appropriate combination.

The composition of the present invention may be administered in a pharmaceutically effective amount. The pharmaceutically effective amount is an amount sufficient to treat a disease at a reasonable benefit/risk ratio applicable to medical treatment, and the effective dose level may be determined according to factors including the patient's health conditions, the type and severity of the disease, drug activity, sensitivity to the drug, administration time, administration route, excretion rate, treatment duration, combined and concurrent drugs, and other factors well known in the medical field. Specifically, it may be 0.1 mg/kg to 100,000 mg/kg per day, more specifically 1 mg/kg to 10,000 mg/kg. The pharmaceutical composition of the present invention may be administered once a day or the daily dose may be divided into several times. Therefore, the above-described dose does not limit the scope of the present invention in any way.

The composition may be administered to mammals such as rats, mice, livestock, and humans by various routes such as parenterally and orally, and all modes of administration may be expected, for example, it may be administered orally, rectally, or by intravenous, intramuscular, subcutaneous, intrauterine, dural, or intracerebroventricular injection.

The pharmaceutical composition of the present invention may further include pharmaceutically acceptable carriers, excipients, diluents, and/or auxiliary components depending on its formulation, method of use, and purpose of use.

**In** addition, the pharmaceutical composition of the present invention may be used alone or in combination with methods using surgery, hormone therapy, drug therapy, and biological response modifiers for the amelioration, alleviation, treatment, or prevention of an inflammatory skin disease.

**In** addition, the present invention provides a quasi-drug composition for improving skin or for preventing or ameliorating an inflammatory skin disease, including CA-SA and magnolol as active ingredients.

As yet another aspect, there is provided a method for improving skin, or a method for preventing or ameliorating an inflammatory skin disease, including a step of applying a quasi-drug composition including CA-SA and magnolol to the skin.

As yet another aspect, there is provided a use of CA-SA and magnolol for preparing a quasi-drug composition for improving skin or for preventing or ameliorating an inflammatory skin disease.

The term "quasi-drug" refers to products that are used for the purpose of diagnosing, treating, ameliorating, alleviating, managing, or preventing human or animal diseases, and have a milder effect than medicines. For example, according to the Pharmaceutical Affairs Act, quasi-drugs are products other than those used for medicinal purposes and include products used for the treatment or prevention of human or animal diseases, and products that have a mild or no direct effect on the human body. For example, a quasi-drug may be selected from the group consisting of disinfecting cleansers, cleaners, kitchen cleaners, cleaning agents, wet wipes, detergents, soaps, hand washes, hair cleansers, hair softeners, and ointments, but is not limited thereto.

**In** the present invention, the CA-SA, magnolol, and the improvement of skin are as described above regarding the cosmetic composition.

**In** the present invention, the formulation method, dose, and further additive components of the medicine and quasi-drug may be appropriately selected from the technologies known in the art.

[Advantageous Effects]

The composition including carnitine salicylate (CA-SA) and magnolol according to the present invention can exhibit excellent skin-improving effects such as acne alleviation, sebum secretion alleviation, antibacterial action, skin erythema alleviation, skin keratin softening, and skin soothing, and can be effectively used for the prevention, amelioration, or treatment of inflammatory skin disease such as acne or atopic dermatitis.

[Description of Drawings]

FIG. 1 shows images confirming the lipid production-inhibiting effect according to Experimental Example 2.
FIG. 2 shows images of the skin of one of the volunteers who were clinically evaluated regarding acne alleviation according to Experimental Example 6.
FIG. 3 shows a graph illustrating the results of the clinical evaluation of acne alleviation according to Experimental Example 6.

[Modes of the Invention]

Hereinafter, the present invention will be described in detail through the following examples. However, the following examples are only intended to illustrate the present invention, and the content of the present invention is not limited to the following examples.

**Examples**

In the experimental examples, carnitine-salicylate (CA-SA) prepared in Preparation Example 1 of Korean Patent Publication No. 10-2022-0126254 was used as a CA-SA eutectic mixture.

**Experimental Example 1. Evaluation of antibacterial activity**

The minimum inhibitory concentrations (MICs) of CA-SA and magnolol against C. *acnes* were measured and compared with an antibiotic (clindamycin) and non-antibiotic agents (benzoyl peroxide and salicylic acid).
C. *acnes* ribotype 5 (strain HL043PA1) was cultured in a reinforced Clostridial broth medium (BD Diagnostics, Franklin Lakes, NJ) in an anaerobic environment (37 °C, 175 rpm). Thereafter, each sample was dissolved in water or dimethyl sulfoxide (DMSO) and serially diluted two-fold, and 7.5 $\mu$L of the resulting solution was added to 142.5 $\mu$L of a bacterial suspension containing $7.5 \times 10^5$ CFU/mL, and incubated for 72 h. Thereafter, turbidity (OD600) was measured (Synergy 2 MultiMode Microplate Reader (BioTek Instrument, Winooski, VT)) to determine the MIC that inhibited bacterial growth by more than 90%. The results are shown in Table 1.

[Table 1]

|  | CA-SA | CA-SA and magnolol (mass ratio = 5:1) | Antibiotic | Non-antibiotic agents | |
|---|---|---|---|---|---|
|  |  |  | Clindamycin | Benzoyl peroxide | Salicylic acid |
| MIC ($\mu$g/ml) | 5000 | 96 | 156.25 | 2500 | 4000-8000 |

As shown in Table 1, when only CA-SA was applied, the MIC for C. *acnes* was 5,000 $\mu$g/ml. However, when CA-SA and magnolol were used in combination, the MIC for C. *acnes* was 96 $\mu$g/ml, indicating that it was more effective in antibacterial action against C. *acnes* compared to when CA-SA was used alone.

Experimental Example 2. Evaluation of sebum secretion alleviation

It was evaluated whether CA-SA and/or magnolol could control sebum secretion by inhibiting lipid production. After confirming that lipid production was effectively induced by applying testosterone, the lipid production inhibition effect was confirmed by applying CA-SA, magnolol, or a combination of the two ingredients.
SZ95 cells, which are primary sebaceous cells, were cultured and maintained in a humidified atmosphere containing 5% $CO_2$ and 95% air at 37 °C. The medium used was Dulbecco's Modified Eagle Medium (DMEM)/Ham's F12 medium (Gibco) supplemented with 10% fetal bovine serum (FBS) and 5 ng/ml recombinant human epidermal growth factor (Invitrogen; Thermo Fisher, Waltham MA). After performing sub-confluent culture, cells were harvested with 0.05% trypsin-ethylenediaminetetraacetic acid (EDTA) (Gibco) and passaged, and cells obtained from the second passage were used.
The cells were seeded at $1 \times 10^5$ cells per well in a 24-well plate (Greiner, Frickenhausen, Germany) and cultured at 37 °C and 5% $CO_2$ for 24 h. Thereafter, the cells were treated with $2 \times 10^{-8}$ M testosterone for 24 h to stimulate lipid production. Thereafter, the cells were treated with 5 $\mu$g/ml CA-SA and/or 0.5 $\mu$g/ml magnolol or vehicle (phosphate-buffered saline (PBS)) for 48 h. The experimental group treated only with testosterone but not treated with CA-SA or magnolol was used as a positive control group. Cells treated with vehicle (PBS) but not treated with testosterone were prepared separately and used as a negative control group.

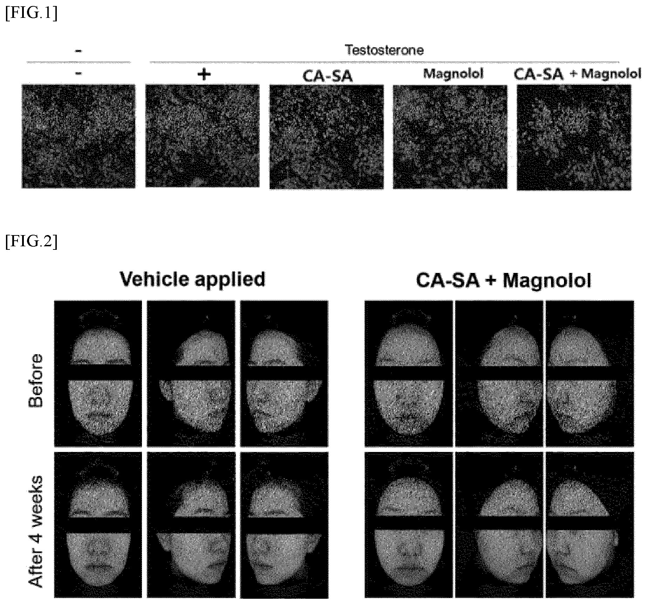

To evaluate the amount of lipid production, the SZ95 cells treated with the sample were washed with PBS, and 4% formaldehyde was added for five minutes at room temperature to fix the cells. 100 $\mu$l of 1 $\mu$g/ml Nile red (Sigma-Aldrich) solution diluted in PBS was added to each well at room temperature for 15 minutes to stain neutral lipids. The results are shown in FIG. 1.

The well plate was covered with aluminum foil to block light, and fluorescence was measured at Ex/Em = 550/580 nm using a microplate reader (BioTek Instruments, Inc.). The percentage value of the absolute fluorescence unit compared to the negative control group was used as the result value. Thereafter, the reduction value of the sample treatment group compared to the positive control group was calculated and is shown in Table 2.

[Table 2]

|  | CA-SA 5 $\mu$g/ml | Magnolol 0.5 $\mu$g/ml | CA-SA 5 $\mu$g/ml + magnolol 0.5 $\mu$g/ml |
|---|---|---|---|
| Reduction value (%) | 5.9 | 3.8 | 23.4 |

When the testosterone-treated SZ95 cells were treated with CA-SA or magnolol, lipid production was reduced by 5.9% and 3.8%, respectively, with no significant difference. However, when CA-SA and magnolol were treated simultaneously, lipid production increased by testosterone treatment was reduced by 23.4%, indicating a significant decrease in lipid production.

Experimental Example 3. Evaluation of the inhibition of inflammation-related factors MMP-1 and VEGF expression

A human epidermal keratinocyte cell line (HaCaT cells) was sub-cultured at 37 °C in a humidified atmosphere containing 5% $CO_2$ and 95% air. The medium used was DMEM containing 10% FBS (Gibco; Thermo Fisher, Waltham MA), penicillin (100 U/ml, Gibco), and streptomycin (100 $\mu$g/100 $\mu$g/ml, Gibco). Separately, the acne pathogen C. *acnes* ribotype 5 (Strain HL043PA1) (1.36 x 10$^9$ CFU/ml, OD = 1.7) was cultured to prepare a C. *acnes* lysate. The C. *acnes* lysate was used to induce the expression of MMP-1 and VEGF in HaCaT cells.

Next, the cultured HaCaT cells were treated with CA-SA and/or magnolol for four hours as shown in Table 3. Thereafter, the C. *acnes* lysate and the medium were mixed at a mass ratio of 3:7, and the resulting mixture was applied to HaCaT cells for 30 minutes. The supernatant was collected, and the concentrations of MMP-1 and VEGF were measured by enzyme-linked immunosorbent assay (ELISA) (R&D Systems Inc., Minneapolis, MN) using the manufacturer's protocol. The untreated experimental group was set as the negative control group, and the percentage values of the MMP-1 and VEGF concentrations of each experimental group compared to the negative control group were used as the result values. The experimental group treated only with the C. *acnes* lysate was set as the positive control group, and the reduction value of the sample treatment group compared to the positive control group was calculated and is shown in Table 3.

In order to confirm the synergistic effect of CA-SA and magnolol, the predicted value (Colby value) and the actual measurement value were compared using the Colby Equation below, and are shown in Table 3.

Colby Equation: E=(A+B)-(A*B/100); E: predicted value; A: efficacy of active ingredient A; B: efficacy of active ingredient B. When the actual measurement value is greater than the predicted value, it is determined that there is a synergistic effect.

[Table 3]

|  | CA-SA 0.1 $\mu$g/ml | Magnolol 4 $\mu$g/ml | CA-SA 0.1 $\mu$g/ml + magnolol 4 $\mu$g/ml | Predicted value (Colby value) |
|---|---|---|---|---|
| MMP-1 reduction value (%) | 49.7 | 33.6 | 81.0 | 66.6 |
| VEGF reduction value (%) | 27.8 | 21.2 | 47.3 | 43.1 |

As shown in Table 3, the expression of MMP-1 and VEGF was effectively inhibited by applying CA-SA and magnolol, and when CA-SA and magnolol were used in combination, significantly superior values were exhibited compared to when they were used alone, confirming that a synergistic effect occurred. From these results, it can be seen that the combined application of CA-SA and magnolol can effectively inhibit the expression of inflammation-related factors under C. *acnes* growth conditions.

**Experimental Example 4. Evaluation of stratum corneum softening efficacy**

To evaluate the softening efficacy on the stratum corneum (SC) of CA-SA and magnolol, a tape stripping test was performed on the SC.

After marking an application site on a volunteer's skin in advance, 50 µl of a solution including CA-SA and/or magnolol at the concentration shown in Table 4 was applied, and after three hours the skin surface was wiped to remove any remaining substances. A solution that included none of the evaluated substances was used as a control. A D-squame adhesive tape disk (CuDerm, Dallas, TX) was attached to the test site, and a cylindrical weight was used to apply a pressure of 150 g/cm$^2$ for three seconds, and then the pressure was removed and the tape was removed from the skin. Three tape strips were obtained from each marked site.

After collecting the tape strips in a polystyrene petri dish (Thermo Fisher), in order to perform keratinolysis quantification, 1 ml of 1 M NaOH was added to each petri dish and the dish was shaken for two hours. Thereafter, 75 µl of the solution was transferred to three wells (96-well plate, Corning), and each was neutralized with an equivalent volume of 1 M HCl. 50 µl of Bradford assay reagent (Bio-Rad, Hercules, CA) was added to each well. After 15 minutes, the optical density at 595 nm (OD 595 nm) was measured using an empty well as a correction value, and the amount of SC protein was quantified using a γ-globulin standard (Bio-Rad). In addition, the relative value (percentage) increase of the experimental group compared to the control group was used as the result value to compare the magnitude of the effect. The results are shown in Table 4.

[Table 4]

|  | CA-SA 5 mg/ml | Magnolol 0.2 mg/ml | **CA-SA** 5 mg/ml + magnolol 0.2 mg/ml |
|---|---|---|---|
| SC exfoliation rate (%) | 64.6 | -22.3 | 123.5 |

As shown in Table 4, when only magnolol was applied, it was difficult to expect the SC exfoliation effect, but when magnolol was applied together with CA-SA, the peeling effect was significantly increased, and the SC exfoliation rate increased by 58.9% compared to treatment with CA-SA alone.

From these results, it can be seen that the combined application of CA-SA and magnolol is more effective in promoting SC peeling than the application of CA-SA and magnolol alone.

**Experimental Example 5. Evaluation of skin erythema recovery through erythema induction inhibition**

To evaluate the erythema recovery efficacy, an erythema index was evaluated before erythema induction, after erythema induction, and 12 days after product use in nine volunteers aged 25 to 35 years. Erythema was induced by applying 1% sodium dodecyl sulfate (SDS) dilution to the forearm for 24 hours, and an emulsion comprising CA-SA 5% by weight of, an emulsion comprising magnolol 0.02% by weight of, an emulsion comprising 5% CA-SA and 0.02% magnolol by weight, and an emulsion containing neither CA-SA nor magnolol(vehicle) was repeatedly applied to the same area twice a day, in the morning and evening, for 12 days.

The erythema index was measured using a Mexameter (MX 18, Courage+Khazaka electronic GmbH, Germany). The erythema index recovery rate was calculated using the following formula, and the effect was compared in terms of the percentage value increase in the erythema index recovery rate of the emulsion application group including each sample relative to the erythema index recovery rate of the vehicle application group (relative recovery rate). The results are shown in Table 5.

$$Skin\ erythema\ index\ recovery_t(\%)$$

$$= \frac{(Erythema\ index_0 - Erythema\ index_t)}{(Erythema\ index_0 - Erythema\ index_{-1})} \times 100$$

*(t: number of days of product use, -1: before inducing erythema,*
*0: immediately after inducing erythema*

In order to confirm the synergistic effect of CA-SA and magnolol, the predicted values (Colby values) and the actual measurement values were compared using the Colby Equation in the same manner as in Experimental Example 3, and the results are shown in Table 5.

[Table 5]

| | CA-SA 5% | Magnolol 0.02% | **CA-SA** 5% + magnolol 0.02% | Predicted value (Colby value) |
|---|---|---|---|---|
| Relative recovery rate (%) | 23.5 | 16.2 | 36.7 | 35.9 |

The erythema index recovery rate compared to the vehicle application group was 23.5% in the CA-SA application group, 16.2% in the magnolol application group, and 36.7% in the simultaneous application group. It can be confirmed that a synergistic effect occurred when CA-SA and magnolol were used in combination, and significantly superior values were exhibited compared to the cases where they were used alone.

**Experimental Example 6. Clinical evaluation of anti-acne activity**

Eight male and female subjects aged 19 to 38 years (average age 25 years) with acne classified as Investigator's Global Assessment (IGA) grade 2 to 3 and Investigator's Static Global Assessment (ISGA) grade 1 or higher were randomly allocated into two groups and evaluated.

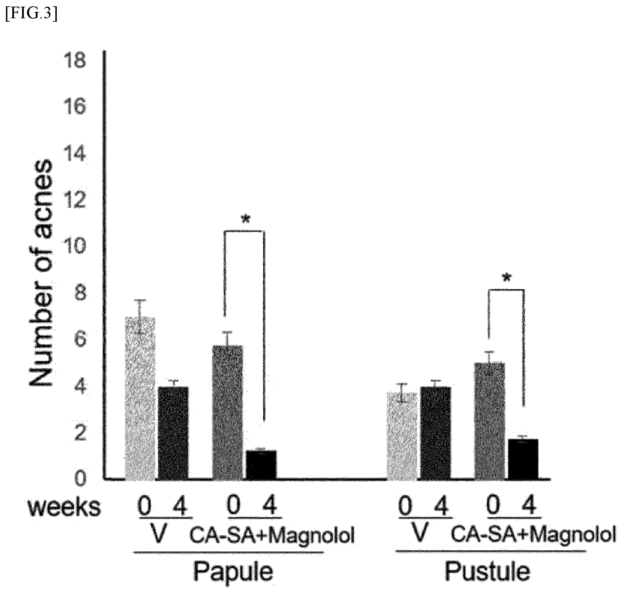

An emulsion including 5% by weight of CA-SA and 0.02% by weight of magnolol and a control vehicle product including no CA-SA were applied every morning and evening, and no other skin care products, ointments, or medications were used on the face during this period. The experiment was conducted for a total of four weeks, and the evaluation was conducted twice, once on the starting day before product application and once after applying the product for four weeks. During the evaluation, all subjects washed the test area only with lukewarm water, and then rested for 20 minutes under constant lighting in the absence of airflow or direct sunlight and under constant temperature and humidity (temperature 22 $\pm$ 2 °C, humidity 50 $\pm$ 5%), and then participated in the evaluation test. The results are shown in FIGS. 2 and 3.

As shown in FIGS. 2 and 3, compared to before product application, the group that applied the emulsion including 5% by weight of CA-SA and 0.02% by weight of magnolol exhibited more alleviation of acne symptoms and a significant decrease in the number of inflammatory acne lesions compared to the group that applied the control vehicle product, and it was confirmed that the applied emulsion was particularly effective for papular and pustular acne.

Experimental Example 7. Sensory evaluation of anti-acne activity

A survey was conducted with 20 subjects with acne to determine whether they felt that their acne had ameliorated when they used a cosmetic including the composition according to the present invention for four weeks. All of the volunteers used the cosmetics they usually used, but additionally used the emulsion containing 5% by weight of CA-SA and 0.02% by weight of magnolol of Experimental Example 6 in the first step after washing their face. The evaluation was scored on a 5-point scale (1: significantly deteriorated, 2: slightly deteriorated, 3: no change, 4: slightly ameliorated, 5: significantly ameliorated), and the results are shown in Table 6.

[Table 6]

| Test item | Average score |
|---|---|
| Reduction in size of pustular and papular acne | 3.7 |
| Reduction in whiteheads and blackheads | 3.8 |
| Reduction in areas inflamed due to acne | 3.8 |
| Increased rate of calming of inflammation due to acne | 4.0 |
| Reduction in sebum secretion | 3.7 |
| Skin calming through acne amelioration | 3.8 |

As shown in Table 6, it was confirmed that when a cosmetic composition including CA-SA and magnolol was repeatedly applied periodically, pustular and papular acne were ameliorated, the areas inflamed due to acne were alleviated and soothed, and sebum secretion was reduced.

**Claims**

**1.** A cosmetic composition for improving skin, comprising carnitine salicylate (CA-SA) and magnolol as active ingredients.

2. The composition of claim 1, wherein the CA-SA is included in an amount of 0.01 to 20 parts by weight based on 100 parts by weight of the total composition.

3. The composition of claim 1, wherein the magnolol is included in an amount of 0.0000001 to 10 parts by weight based on 100 parts by weight of the total composition.

4. The composition of claim 1, wherein the weight ratio of the CA-SA and magnolol is 500000 to 20:1.

5. The composition of claim 1, wherein improving skin is one or more selected from the group consisting of acne alleviation, sebum secretion alleviation, skin soothing, antibacterial action, skin erythema alleviation, acne inflammation amelioration, and promotion of keratin peeling.

6. A pharmaceutical composition for preventing or treating an inflammatory skin disease, comprising carnitine salicylate (CA-SA) and magnolol as active ingredients.

7. The composition of claim 6, wherein the inflammatory skin disease is one or more selected from the group consisting of atopic dermatitis, seborrheic dermatitis, and acne.

8. A quasi-drug composition for skin improvement, comprising carnitine salicylate (CA-SA) and magnolol as active ingredients.

9. The composition of claim 8, wherein the skin improvement is one or more selected from the group consisting of acne alleviation, sebum secretion alleviation, skin soothing, antibacterial action, skin erythema alleviation, acne inflammation amelioration, and keratin peeling promotion.

10. A quasi-drug composition for preventing or ameliorating an inflammatory skin disease, comprising carnitine salicylate (CA-SA) and magnolol as active ingredients.

11. The composition of claim 10, wherein the inflammatory skin disease is one or more selected from the group consisting of atopic dermatitis, seborrheic dermatitis, and acne.

12. A method for improving skin, comprising a step of applying a composition including carnitine salicylate (CA-SA) and magnolol to the skin.

13. A method for preventing or treating an inflammatory skin disease, comprising a step of administering a composition including carnitine salicylate (CA-SA) and magnolol to a subject in need thereof.

[FIG.1]

[FIG.2]

[FIG.3]

## INTERNATIONAL SEARCH REPORT

| | International application No. |
|---|---|
| | **PCT/KR2023/021439** |

| A. | CLASSIFICATION OF SUBJECT MATTER |
|---|---|

**A61K 8/365**(2006.01)i; **A61K 8/34**(2006.01)i; **A61Q 19/00**(2006.01)i

According to International Patent Classification (IPC) or to both national classification and IPC

| B. | FIELDS SEARCHED |
|---|---|

Minimum documentation searched (classification system followed by classification symbols)

A61K 8/365(2006.01); A61K 31/05(2006.01); A61K 8/34(2006.01); A61K 8/368(2006.01); A61K 8/44(2006.01); A61K 8/97(2006.01); A61Q 17/00(2006.01); A61Q 19/00(2006.01); A61Q 19/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 카르니틴살리실레이트(carnitine salicylate, CA-SA), 마그놀롤(magnolol), 피부 (skin)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2022-0126254 A (LG H&H CO., LTD.) 15 September 2022 (2022-09-15)<br>See claims 1-11; and paragraphs [0129]-[0130]. | 1-12 |
| Y | KR 10-2006-0014203 A (BIOSPECTRUM, INC.) 15 February 2006 (2006-02-15)<br>See abstract; claims 1-10; and paragraphs [0007], [0016] and [0051]. | 1-12 |
| Y | KR 10-1221475 B1 (LEE, Sung Nack) 15 January 2013 (2013-01-15)<br>See abstract; claims 1-4; and paragraph [0002]. | 1-12 |
| Y | KR 10-0699538 B1 (BIOSPECTRUM, INC.) 23 March 2007 (2007-03-23)<br>See claims 1-4; and pages 3 and 5. | 1-12 |
| A | KR 10-2010-0060711 A (AMOREPACIFIC CORPORATION) 07 June 2010 (2010-06-07)<br>See entire document. | 1-12 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 April 2024** | **12 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/021439**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **13**
   because they relate to subject matter not required to be searched by this Authority, namely:

   Claim 13 pertains to a method for treatment of the human body by surgery or therapy, and thus pertains to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/KR2023/021439**

| Patent document cited in search report | | | Publication date (day/month/year) | Patent family member(s) | | | Publication date (day/month/year) |
|---|---|---|---|---|---|---|---|
| KR | 10-2022-0126254 | A | 15 September 2022 | CN | 116981438 | A | 31 October 2023 |
| | | | | EP | 4306100 | A1 | 17 January 2024 |
| | | | | WO | 2022-191602 | A1 | 15 September 2022 |
| KR | 10-2006-0014203 | A | 15 February 2006 | | None | | |
| KR | 10-1221475 | B1 | 15 January 2013 | KR | 10-2011-0063949 | A | 15 June 2011 |
| KR | 10-0699538 | B1 | 23 March 2007 | KR | 10-2007-0031094 | A | 19 March 2007 |
| KR | 10-2010-0060711 | A | 07 June 2010 | KR | 10-1017586 | B1 | 28 February 2011 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20220126254
- KR 1020220126254